**Europäisches Patentamt**

**European Patent Office**

**Office européen des brevets**

(11) Veröffentlichungsnummer: **0 021 343**
**A1**

(12) # EUROPÄISCHE PATENTANMELDUNG

(21) Anmeldenummer: **80103405.9**

(51) Int. Cl.³: **A 61 F 5/44**

(22) Anmeldetag: **19.06.80**

(30) Priorität: **21.06.79 DE 7917734 U**

(43) Veröffentlichungstag der Anmeldung: **07.01.81**
**Patentblatt 81/1**

(84) Benannte Vertragsstaaten: **AT BE CH DE FR GB IT LI LU NL SE**

(71) Anmelder: **Intermedicat GmbH, Gerliswilstrasse 45, CH-6020 Emmenbrücke (CH)**

(72) Erfinder: **Schlaack, Uwe, Söhrestrasse 15, D-3501 Körle (DE)**

(74) Vertreter: **von Kreisler, Alek et al, Deichmannhaus am Hauptbahnhof, D-5000 Köln 1 (DE)**

(54) **Anus-praeter-Beutel.**

(57)  Die Anmeldung betrifft einen Anus-praeter-Beutel (1) insbesondere für Pelotten, aus flexiblem Kunststoffmaterial mit einem geschlossenen und einem offenen Ende (3, 2). Der Beutel (1) ist als nahtloser Beutel aus PVC ausgebildet.

Intermedicat GmbH., Emmenbrücke, Schweiz     0021343

Anus-praeter-Beutel

Die Erfindung betrifft einen Anus-praeter-Beutel aus
flexiblem Kunststoffmaterial.

Anus-praeter-Beutel werden für Patienten mit künstlichem
Darmausgang verwendet.

Patienten mit künstlichem Darmausgang haben grundsätzlich
die Wahl zwischen einer geklebten, geruchsdichten Versorgung und einer weniger geruchsdichten Versorgung durch
Beutel, die mit Gürtel getragen werden.

Es gibt verschiedene Gründe, weshalb ein Ostomie-Patient
nicht die geruchsdichte Klebe-Versorgung verwenden kann.
Hier spielen einmal die Hautprobleme eine Rolle und zum
anderen auch die physiologische Situation (Narben, Bruch).
Auch ist es üblich,daß Patiente, welche die bessere Klebe-
Versorgung einsetzen, von Zeit zu Zeit ihrer Haut Erholung
gönnen müssen und dann auf die Gürtel Versorgung zurückgreifen.

Die Ausstattung von Pelotten-Gürteln mit geruchsdichten
Beuteln stößt aus technischen Gründen auf folgende Schwierigkeite: die geruchsdichte Folie ist dicker und sperriger
als die üblicherweise bei Pelottenbeuteln verwendete Polyäthylenfolie. Da die Beutel aus Folie geschweißt werden,
bildensich Nahtstellen, die bei dem geruchsdichten  Material
besonders hart und sperrig sind. Diese Nahtstellen drücken
die Haut, verursachen Schmerzen und erzeugen Undichtigkeiten beim Abschluss auf der Haut.

Es besteht daher ein Bedürfnis, einen Anus-praeter-Beutel
zu schaffen, der die genannten Nachteile nicht aufweist.

Demgemäss stellt sich die Erfindung die Aufgabe, einen
Anus-praeter-Beutel, der insbesondere für Pelotten geeignet
ist, aus flexiblem Kunststoffmaterial mit einem geschlossenen und einem offenen Ende zu schaffen, der die obigen Nachteile nicht aufweist,und dem Bedürfnis gerecht wird. Die Aufgabe wird durch den erfindungsgemässen Anus-praeter-Beutel, insbesondere für Pelotten, aus flexiblem Kunststoffmaterial mit einem geschlossenen und einem offenen Ende gelöst, der dadurch gekennzeichnet ist, daß er als nahtloser Beutel aus Polyvinylchlorid (PVC) ausgebildet ist.

Der erfindungsgemässe Anus-praeter-Beutel wird nach dem Tauchverfahren hergestellt.

Der erfindungsgemässe getauchte Beutel bietet gegenüber den bekannten geschweißten Beuteln viele Vorteile. Durch das Tauchverfahren hat er keine Nahtstellen wie ein geschweißter Beutel. Der erfindungsgemässe Beutel ist durch die Verwendung von Polyvinylchlorid als Material absolut geruchsdicht. Er ist außerdem natürlich auch geruchsdicht, weil beim Kontakt mit der Haut keine Nahtstellen den Austritt von Gasen bzw. Exkrementen erlauben.

Der erfindungsgemässe Beutel aus PVC-Material hat ferner den Vorteil, daß er absolut knister- und raschelfrei ist, was das unauffällige Tragen des Beutels durch die Ostomie-patienten erleichtert. Weiterhin hat er im Gegensatz zu den geschweißten Beuteln keine scharfen Kanten und bietet psychologisch den Ostomie-Patienten das Gefühl größerer Sicherheit, weil die Schweißnähte fehlen.

Schließlich ist die Herstellung im Vergleich zu den bisher üblichen geruchsdichten Klebebeuteln in der Anwendung für den Patienen wesentlich billiger.

Bei einer bevorzugten Ausführungsform des erfindungsgemässen Anus-praeter-Beutels ist der offene Randteil des Beutels mit einer aufgerollten, umlaufenden Randverstärkung versehen Dies verhindert ein Ausfransen des Beutelmaterials an seinem offenen Ende.

Vorzugsweise ist das geschlossene Ende des erfindungsgemässen Anus-praeter-Beutels abgerundet und der Beutel·
erweitert sich zm offenen Ende hin.

Vorzugsweise weist der erfindungsgemässe Beutel flachgedrückte Form auf.

Die Erfindung wird imFolgenden durch die Beschreibung der
Zeichnung weiter erläutert.

Der in Draufsicht dargestellte erfindungsgemässe Anus-
praeter-Beutel 1 weist ein offenes Ende 2 und ein geschlossenes Ende 3 auf. Er ist nahtlos und aus Polyvinylchlorid hergestellt. Das geschlossene Ende 3 des
Beutels 1 ist abgerundet und hat etwa eine halbkreisförmige Form. Bis zur Hälfte seiner Gesamtlänge geht
der Beutel 1 vom geschlossenen Ende aus in einen im
wesentlichen parallelwandigen Teil 4 über. An diesen
schließt sich nahtlos ein sich zum offenen Ende erweiternder Teil 5 an, der eine konische Form aufweist. Am
offenen Ende 2 des Beutels 1 ist der offene Randteil
mit einer umlaufenden Randverstärkung 6 versehen, die
durch Aufrollen des PVC-Materials am offenen Ende 2 gebildet worden ist.

Die Herstellung der erfindungsgemässen Anus-praeter-Beutel erfolgt nach dem üblichen Tauchverfahren, das dem
Fachmann bekannt ist. Beispielsweise wird dieses Tauchverfahren zur Herstellung von nahtlosen Einmalhandschuhen
verwendet.

P a t e n t a n s p r ü c h e
------------------------------

1.   Anus-praeter-Beutel (1), insbesondere für Pelotten,
aus flexiblem Kunststoffmaterial mit einem geschlossenen
und einem offenen Ende (3, 2), dadurch gekennzeichnet,
daß er als nahtloser Beutel aus PVC ausgebildet ist.

2.   Anus-praeter-Beutel nach Anspruch 1, dadurch gekennzeichnet, daß der offene Randteil (2) des Beutels
(1) mit einer aufgerollten, umlaufenden Randverstärkung (6) versehen ist.

3.   Anus-praeter-Beutel nach Ansprüchen 1 und 2, dadurch gekennzeichnet, daß das geschlossene Ende (3)
des Beutels (1) abgerundet ist und daß der Beutel (1)
sich zum offene Ende hin erweitert (5).

4.   Anus-praeter-Beutel nach Ansprüchen 1 - 3, dadurch gekennzeichnet, daß der Beutel (1) flachgedrückte
Form aufweist.

0021343

**Europäisches Patentamt**

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| | DE - A - 1 918 876 (PHARMA-PLAST A/S) <br> * Anspruch * | 1 |
| | GB - A - 1 277 432 (P.F. BULLEN) <br> * Seite 1, Zeilen 9 bis 12; Fig. 1 * | 1 |
| A | DE - A1 - 2 345 227 (H.K. BIELICK) <br> * Ansprüche 1, 3, 9; Fig, 2, 3 * | 1 |
| A | FR - A - 1 113 210 (E.-R. TUDES) <br> * Fig. 1 * | 1-4 |
| A | US - A - 1 656 328 (T.J. LE CRAS) <br> * Fig. 1, 2 * | 1,2,4 |
| A | US - A - 2 527 321 (N.H. METTE) <br> * Fig. 1 * | 1,2 |
| A | US - A - 2 549 649 (D.H. VAN HOVE) <br> * Fig. 1 bis 3 * | 1 |

**KLASSIFIKATION DER ANMELDUNG (Int.Cl.3)**

A 61 F 5/44

**RECHERCHIERTE SACHGEBIETE (Int. Cl.3)**

A 61 F 5/00

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

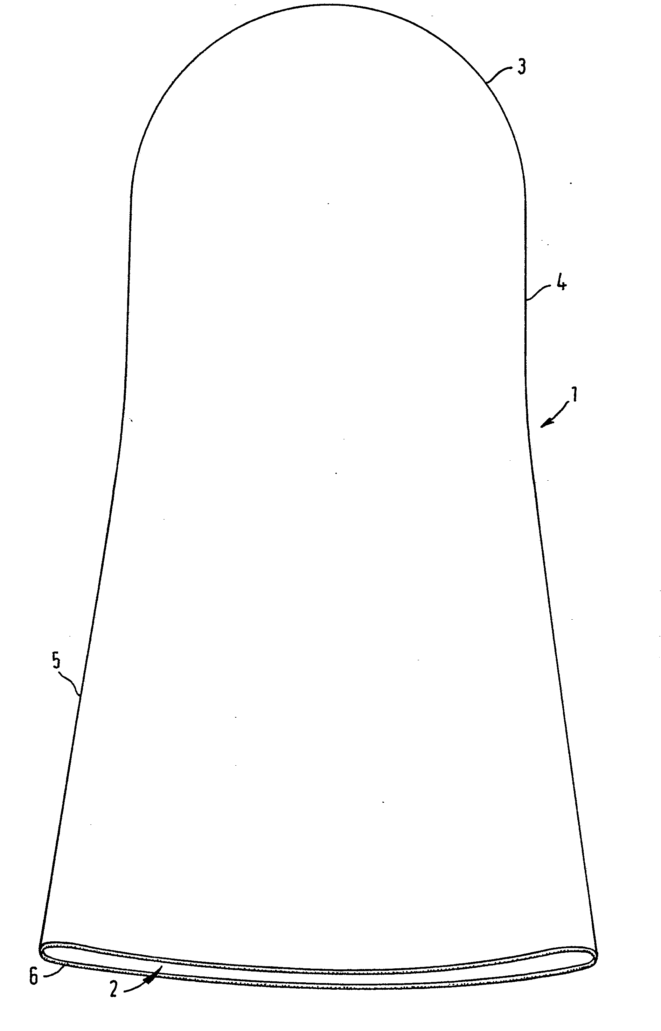

| Recherchenort | Abschlußdatum der Recherche | Prüfer |
|---|---|---|
| Berlin | 17-09-1980 | KANAL |

EPA form 1503.1 06.78